# EUROPEAN PATENT APPLICATION

(11) **EP 4 268 818 A1**
(43) Date of publication of application: **01.11.2023**
(21) Application number: 21909544.5
(22) Date of filing: 24.12.2021
(51) Int. Cl.: A61K 31/438, A61P 1/08, C07D 471/10

(54) **USE OF NK1 ANTAGONIST PRODRUG COMPOUND IN COMBINATION WITH 5-HT3 RECEPTOR ANTAGONIST**

(30) Priority: 25.12.2020 CN 202011559059; 13.09.2021 CN 202111066727
(71) Applicant: Shanghai Shengdi Pharmaceutical Co., Ltd, Shanghai 201210 (CN); Shanghai Senhui Medicine Co., Ltd., Shanghai 201203 (CN); Jiangsu Hengrui Pharmaceuticals Co., Ltd., Lianyungang, Jiangsu 222047 (CN)
(72) Inventor: XU, Daping, Shanghai 201210 (CN); YANG, Changyong, Lianyungang, Jiangsu 222047 (CN); LIAO, Cheng, Lianyungang, Jiangsu 222047 (CN); ZHANG, Lianshan, Lianyungang, Jiangsu 222047 (CN); HUANG, Jian, Shanghai 201203 (CN)
(74) Representative: Regimbeau
(86) International application number: PCT/CN2021/141009
(87) International publication number: WO 2022/135549

(57) **Abstract**

The present disclosure relates to the use of an NK1 antagonist prodrug compound and a 5-HT3 receptor antagonist. In particular, the present disclosure relates to the use of a compound represented by formula (I) or a pharmaceutically acceptable salt thereof in combination with a 5-HT3 receptor antagonist in the preparation of a drug for preventing or treating nausea and/or vomiting.

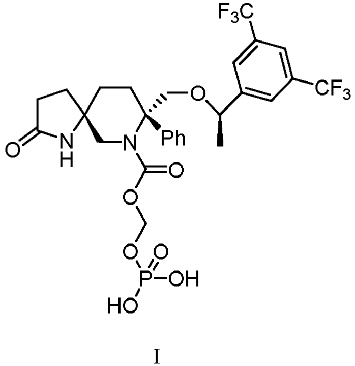

## Description

### TECHNICAL FIELD

The present disclosure relates to the field of pharmaceuticals, and particularly relates to use of a rolapitant prodrug in combination with a 5-HT3 receptor antagonist in the preparation of a medicament for preventing or treating nausea and/or vomiting.

### BACKGROUND

Tachykinins are peptide ligands of neurokinin receptors. Neurokinin receptors, such as NK1, NK2, and NK3, are involved in various biological processes. They can be found in the nervous and circulatory systems of mammals and in surrounding tissues. Thus, the regulation of such receptors has been studied for potential treatment or prevention of various diseases in mammals. Typical neurokinin receptor antagonists and use thereof include: US5760018 (1998) (pain, inflammation, migraine, and vomiting), US5620989 (1997) (pain, nociception, and inflammation), WO95/19344 (1995), WO94/13639 (1994), and WO94/10165 (1994). Other classes of NK1 receptor antagonists also include: Wu et al., Tetrahedron 56, 3043-3051 (2000); Rombouts et al., Tetrahedron Letters 42, 7397-7399 (2001); and Rogers et al., Tetrahedron 57, 8971-8981 (2001).

US7049320 provides an effective and selective NK1 antagonist with beneficial therapeutic and pharmacological properties and good metabolic stability, rolapitant, which may be in the form of a free base or in the form of a pharmaceutically acceptable salt, and is suitable for a formulation for parenteral administration, 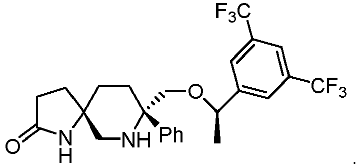 US9101615 provides a prodrug of rolapitant, i.e., a prodrug and a salt thereof of the compound of formula I with the hydrogen of free amines (or two amines) replaced by a group selected from the group consisting of -Y and -X, wherein Y is selected from the group consisting of -P(O)(OH)₂, -S(O)ₙ₁R¹, -C(O)(C₁₋₆ alkyl)X, -C(O)(C₁₋₆ alkyl)(aryl), and -C(O)OR⁴; X is selected from the group consisting of -NR²R³, -P(O)(OH)₂, and -S(O)ₙ₁R₁; R¹ is H or C₁₋₆ alkyl; R² is H or C₁₋₆ alkyl; R³ is H or C₁₋₆ alkyl; R⁴ is H or C₁₋₆ alkyl; and n1 is 0-4. The prodrug can be used in suitable liquid formulations (with or without the parenteral delivery vehicle) to treat patients in need of treatment thereof.

In another aspect, the drug-induced hemolysis is caused by the destruction of a large number of red blood cells due to immune factors after the drug enters the human body, and hemolysis phenomena such as anemia, jaundice, and soy sauce-colored urine appear clinically. The drug-induced hemolytic anemia can be classified into three types: (1) drug-induced immunity, leading to an antibody-mediated hemolytic reaction; (2) drug actions on red blood cells with genetic enzyme deficiencies (e.g., G6PD deficiency); and (3) hemolytic reaction of the drug to abnormal hemoglobin. The key to treating the disease is to stop taking the relevant drugs and control the occurrence of hemolysis so as to prevent the occurrence of complications. In order to solve the problem of low solubility of the compound of formula I at physiological pH, a co-solvent-based formulation containing Captisol, propylene glycol, and ethanol was used to significantly increase the solubility of compound 1. However, the co-solvent formulation had a significant hemolytic effect after intravenous administration. CN102573475 discloses an improved formula containing polyethylene glycol 15-hydroxystearate and medium chain triglyceride. However, the hemolytic effect of the pharmaceutical composition is still not completely addressed, even if the compound of formula I is prepared as a phosphate-containing prodrug.

PCT/CN2020/098460 provides a novel NK1 antagonist prodrug compound effective in treating various physiological disorders, conditions, and diseases with fewer side effects, the structure of which is shown below:

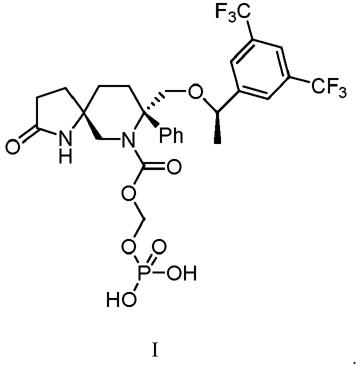

The present disclosure provides use of a novel NK1 antagonist prodrug compound effective in treating various physiological disorders, conditions, and diseases with reduced side effects in combination with a 5-HT3 receptor antagonist in the preparation of a medicament for preventing or treating nausea and/or vomiting, which shows good therapeutic effects.

### SUMMARY

The present disclosure provides use of a compound of formula (I) or a pharmaceutically acceptable salt thereof in combination with a 5-HT3 receptor antagonist in the preparation of a medicament for preventing or treating nausea and/or vomiting,

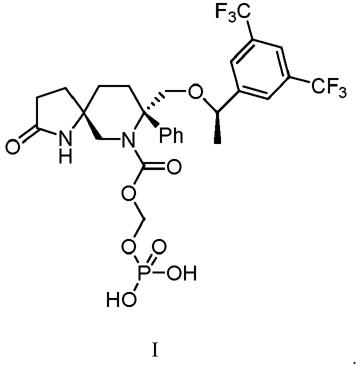

In certain embodiments, the 5-HT3 receptor antagonist is selected from the group consisting of granisetron, ondansetron, ramosetron, tropisetron, palonosetron, and dolasetron, preferably palonosetron.

In certain embodiments, the compound of formula (I) or the pharmaceutically acceptable salt thereof is administered at a dose of 10-500 mg, e.g., 10 mg, 20 mg, 27.25 mg, 30 mg, 40 mg, 50 mg, 54.5 mg, 60 mg, 70 mg, 80 mg, 81.75 mg, 90 mg, 100 mg, 109 mg, 110 mg, 120 mg, 130 mg, 136.25 mg, 140 mg, 150 mg, 160 mg, 163.5 mg, 170 mg, 180 mg, 190 mg, 190.75 mg, 200 mg, 210 mg, 218 mg, 220 mg, 230 mg, 240 mg, 245.25 mg, 250 mg, 260 mg, 270 mg, 272.5 mg, 280 mg, 290 mg, 299.75 mg, 300 mg, 310 mg, 320 mg, 327 mg, 330 mg, 340 mg, 350 mg, 354.25 mg, 360 mg, 370 mg, 380 mg, 381.5 mg, 390 mg, 400 mg, 408.75 mg, 410 mg, 420 mg, 430 mg, 436 mg, 440 mg, 450 mg, 460 mg, 463.25 mg, 470 mg, 480 mg, 490 mg, 490.5 mg, or 500 mg, and may be administrated at a frequency of once a day, twice a day, three times a day, once a week, or once every two weeks.

In certain embodiments, the 5-HT3 receptor antagonist is administered at a dose of 0.075-1 mg, e.g., 0.125 mg, 0.25 mg, 0.375 mg, 0.5 mg, 0.625 mg, or 0.75 mg, preferably 0.25-0.75 mg, and may be administrated at a frequency of once a day, twice a day, three times a day, once a week, or once every two weeks.

In certain embodiments, the compound of formula (I) or the pharmaceutically acceptable salt thereof is administered at a dose of 27.25 mg, 54.5 mg, 81.75 mg, 109 mg, 136.25 mg, 163.5 mg, 190.75 mg, 218 mg, 245.25 mg, 272.5 mg, 299.75 mg, 327 mg, 354.25 mg, 381.5 mg, 408.75 mg, 436 mg, 463.25 mg, or 490.5 mg, and may be administrated at a frequency of once a day, twice a day, three times a day, once a week, or once every two weeks. The 5-HT3 receptor antagonist may be administered at a dose of 0.125 mg, 0.25 mg, 0.375 mg, 0.5 mg, 0.625 mg, or 0.75 mg, and may be administrated at a frequency of once a day, twice a day, three times a day, once a week, or once every two weeks.

The pharmaceutically acceptable salt of the drug described herein may be hydrochloride, phosphate, hydrophosphate, sulfate, hydrosulfate, sulfite, acetate, oxalate, malonate, valerate, glutamate, oleate, palmitate, stearate, laurate, borate, p-toluenesulfonate, methanesulfonate, isethionate, or maleate, malate, tartrate, benzoate, pamoate, salicylate, vanillate, mandelate, succinate, gluconate, lactobionate, lauryl sulfonate, or the like.

The method described herein is used for treating or preventing nausea and/or emesis caused by a variety of events, including chemotherapy-induced nausea and vomiting ("CINV") caused by moderately emetogenic chemotherapy or highly emetogenic chemotherapy, radiation-induced nausea and vomiting ("RINV"), and postoperative nausea and vomiting ("PONV"). The method is preferably performed shortly before the occurrence of a vomiting-inducing event (i.e., no more than 1 or 2 h before the event). The method can be used for treating nausea and/or vomiting during the acute phase or the delayed phase of nausea. The mode of administration of the combination described herein is selected from the group consisting of simultaneous administration, separate formulation and co-administration, and separate formulation and sequential administration.

The route of administration of the combination described herein is selected from oral administration, parenteral administration (including but not limited to intravenous injection, subcutaneous injection, and intramuscular injection), and transdermal administration.

The present disclosure further relates to a method for preventing or treating nausea and/or vomiting, which comprises administering to a patient the compound of formula (I) or the pharmaceutically acceptable salt thereof and the 5-HT3 receptor antagonist.

The present disclosure further relates to a method for preventing or treating vomiting, which comprises administering to a patient suffering from or at risk of suffering from vomiting the combination of the present disclosure. In other embodiments, the present disclosure provides a method for preventing or treating vomiting by administering one or more of the combinations described herein. The combination is preferably administered shortly before a vomiting-inducing event (i.e., no more than 2 h before the event). The vomiting may be acute vomiting (i.e., vomiting occurring within about 24 h after a vomiting-inducing event), or delayed emesis (i.e., vomiting occurring after the acute phase but within 7, 6, 5, or 4 days after a vomiting-inducing event). The vomiting may include chemotherapy-induced nausea and vomiting ("CINV") caused by moderately emetogenic chemotherapy or highly emetogenic chemotherapy, radiation-induced nausea and vomiting ("RINV"), and postoperative nausea and vomiting ("PONV"). In the embodiments of the present disclosure, the combination optionally further comprises other components, including but not limited to other antiemetics and the like.

The present disclosure further provides a compound of formula (I) or a pharmaceutically acceptable salt thereof for use in the prevention or treatment of nausea and/or vomiting, in combination with a 5-HT3 receptor antagonist.

The present disclosure further provides a 5-HT3 receptor antagonist for use in the prevention or treatment of nausea and/or vomiting, in combination with a compound of formula (I) or a pharmaceutically acceptable salt thereof.

The present disclosure further relates to a pharmaceutical composition comprising the compound of formula (I) or the pharmaceutically acceptable salt thereof, the 5-HT3 receptor antagonist, and one or more pharmaceutically acceptable carriers. The pharmaceutical composition may be formulated into any pharmaceutically acceptable dosage form, For example, it may be formulated into a tablet, a capsule, a pill, a granule, a solution, a suspension, a syrup, an injection (including a solution for injection, a sterile powder for injection, and a concentrated solution for injection), a suppository, an inhalant, or a spray.

The pharmaceutical composition comprising the compound of formula (I) or the pharmaceutically acceptable salt thereof, and the 5-HT3 receptor antagonist described herein may be administered alone or in combination with one or more therapeutic agents. The components to be combined (e.g., a compound of formula (I) or a pharmaceutically acceptable salt thereof, a 5-HT3 receptor antagonist, and any other component drug) may be administered simultaneously or separately and sequentially. Furthermore, the components to be combined may also be administered in combination in the same formulation or in separate and distinct formulations.

The present disclosure further provides a pharmaceutical package in which the pharmaceutical composition comprising the compound of formula (I) or the pharmaceutically acceptable salt thereof and the 5-HT3 receptor antagonist described herein is packaged.

The term "combination" as used herein is a mode of administration and refers to the administration of at least one dose of the compound of formula (I) or the pharmaceutically acceptable salt thereof and at least one dose of the chemotherapeutic agent over a certain period of time, wherein the agents administered all exhibit pharmacological effects. The period of time may be within one administration cycle, preferably within 4 weeks, within 3 weeks, within 2 weeks, within 1 week, or within 24 h, more preferably within 12 h. The compound of formula (I) or the pharmaceutically acceptable salt thereof and the 5-HT3 receptor antagonist may be administered simultaneously or sequentially. A treatment in which the compound of formula (I) or the pharmaceutically acceptable salt thereof and the 5-HT3 receptor antagonist are administered by the same route of administration or different routes of administration is included within the period of time. The mode of administration of the combination described herein is selected from the group consisting of simultaneous administration, separate formulation and co-administration, and separate formulation and sequential administration.

The term "postoperative nausea and/or vomiting" (PONV) has conventional meaning in the art. It is well known in the art that PONV means that one or more episodes of vomiting (vomiting and/or retching) or the desire to vomit (nausea) occurs after surgery. Retching involves the same physiological mechanisms as vomiting, but occurs at a closed glottis. PONV may be defined as nausea and/or vomiting occurring within 48 h after the end of surgery, or may be defined as nausea and/or vomiting occurring within 24 h after the end of surgery.

"Therapeutically effective amount" refers to the amount of a therapeutic agent that produces the desired effect for which it is administered. In some embodiments, the term refers to an amount sufficient to treat a disease, disorder and/or condition when administered to a population suffering from or susceptible to such a disease, disorder and/or condition according to a regimen. In some embodiments, the therapeutically effective amount is an amount that reduces the incidence and/or severity, and/or delays the onset of, one or more symptoms of a disease, disorder, and/or condition. It will be understood by those of ordinary skill in the art that the term "therapeutically effective amount" is not actually required to achieve successful treatment in a particular individual. Conversely, the therapeutically effective amount may be an amount that, when administered to a patient in need of such treatment, provides a particular desired pharmacological response in a large number of subjects. In some embodiments, reference to the therapeutically effective amount can refer to an amount as measured in one or more particular tissues (e.g., tissues affected by a disease, disorder, or condition) or fluids (e.g., blood, saliva, serum, sweat, tears, urine, etc.). It will be understood by those of ordinary skill in the art that, in some embodiments, the therapeutically effective amount of a particular agent or therapy may be formulated and/or administered in a single dose. In some embodiments, a therapeutically effective agent may be formulated and/or administered in multiple doses, e.g., as part of a regimen.

All numbers herein may be understood as being modified by "about", which, when referring to a measurable value such as an amount and time of duration, is intended to encompass a variation of ±10%, preferably ±5%, or more. Unless otherwise stated, such variations are appropriate to obtain the drug at the desired amount, preferably ± 1%, even more preferably ± 0.1% from the specified value.

The present disclosure improves the therapeutic effects of nausea and/or vomiting by administering the compound of formula (I) or the pharmaceutically acceptable salt thereof in combination with the 5-HT3 receptor antagonist.

### DETAILED DESCRIPTION

The present disclosure is further described below with reference to examples, which are not intended to limit the scope of the present disclosure.

Experimental procedures without conditions specified in the examples of the present disclosure are generally conducted according to conventional conditions, or according to conditions recommended by the manufacturer of the starting materials or commercial products. Reagents without specific origins indicated are commercially available conventional reagents.

### Example 1.

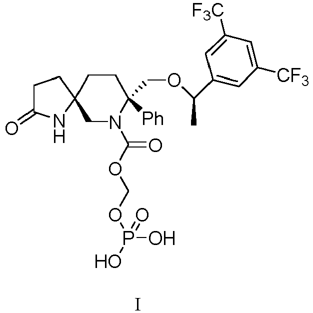

### Step 1

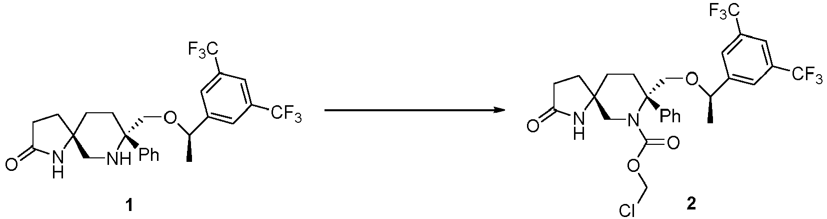

Under N₂ atmosphere, compound 1 (2.43 g, 4.86 mmol, 1 eq) was weighed, added to a 100 mL three-necked flask, and dissolved in dichloromethane (36 mL), and diisopropylethylamine (5 g, 38.76 mmol, 8 eq) was added. The mixture was cooled to -30 °C, and trimethylchlorosilane (1.36 g, 12.52 mmol, 2.6 eq) was added. The resulting mixture was stirred at room temperature for 2 h. Then, the reaction solution was cooled to -25 °C and a solution of chloromethyl chloroformate (0.77 g, 6 mmol, 1.23 eq) in dichloromethane was added. The resulting mixture was stirred until the reaction was completed, with the temperature controlled to be -20 °C to -5 °C. The reaction solution was poured into ice water for liquid separation, and the aqueous phase was extracted with dichloromethane. Water and 1 N hydrochloric acid solution were added for liquid separation, and the organic phase was washed sequentially with brine, saturated aqueous sodium bicarbonate solution, and brine, dried over anhydrous sodium sulfate, filtered, and concentrated to obtain a yellow jelly (3.0 g, yield: 104%).

### Step 2

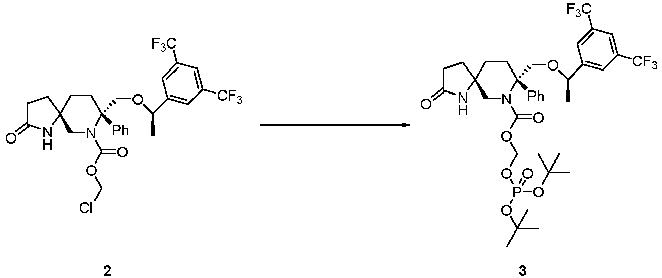

Under N₂ atmosphere, compound 2 (2.8 g, 4.53 mmol, 1 eq), tetrabutylammonium iodide (1.68 g, 4.55mmol, 1 eq), potassium di-*tert*-butyl phosphate (5.63 g, 22.67mmol, 5 eq), and dioxane (84 mL) were added to a 500 mL three-necked flask. The mixture was heated to 55 °C and stirred for 4 h. The reaction solution was cooled and poured into ethyl acetate and water for liquid separation, and the aqueous phase was extracted with ethyl acetate. The organic phase was washed with an aqueous sodium sulfite solution, then washed sequentially with water and brine, dried over anhydrous sodium sulfate, filtered, and concentrated to obtain a yellow foam (3.73 g, yield: 107%).

### Step 3

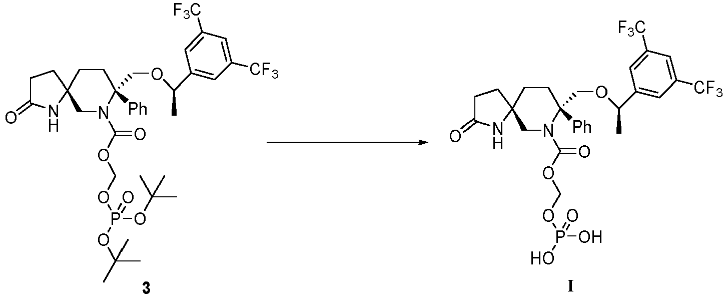

Under N₂ atmosphere, compound 3 (6.65 g, 8.67 mmol, 1 eq) was added to a 500 mL single-necked flask and dissolved in dichloromethane (200 mL), and trifluoroacetic acid (9.89 g, 86.7 mmol, 10.0 eq) was slowly added in an ice-water bath. The mixture was stirred until the reaction was completed. The reaction solution was concentrated to obtain an oil (2.29 g), which was purified by using a reverse-phase silica gel column (C18) (solution A: 20 mmol NH₄HCO₃ aqueous solution, solution B: acetonitrile), adjusted to pH 1-2 with 1 M phosphoric acid, and extracted with dichloromethane. The organic phase was washed with saturated brine, dried over anhydrous sodium sulfate, filtered, and concentrated to obtain the target product, i.e., the compound of formula (I) (2.7 g).

Example 2. Toxicity Test of the Compound of Formula (I) in Combination with Palonosetron in Rats

The compound of formula (I), palonosetron, or the compound of formula (I) + palonosetron was intravenously injected into rats 1-2 times a week in 29 consecutive days, followed by interruption for recovery for 4 weeks. The nature, the degree, the dose-effect and time-effect relationships, and the reversibility of possible toxic reactions were observed, the target organ or tissue of toxicity was determined, and the toxicokinetic characteristics of the agent were studied to understand the relation between the exposure dose and the toxicological result in the toxicity research.

### Drug preparation

After the conversion by content, the desired amount of the compound of formula (I) was weighed and prepared to the desired concentration by adding a proper amount of 0.02 M phosphate buffer (about pH 7.40). A low-concentration formulation for administration can be obtained by diluting a high-concentration formulation for administration, and the prepared formulation is used after being filtered (through a PES filter membrane). Temporary storage conditions and validity period for the prepared formulation: 15-25 °C, protected from the light, available within 72 h.

After the conversion by content, the desired amount of palonosetron was weighed and prepared to the desired concentration by adding a proper amount of 0.02 M phosphate buffer (about pH 7.40). A low-concentration formulation for administration can be obtained by diluting a high-concentration formulation for administration, and the prepared formulation is used after being filtered (through a PES filter membrane). Temporary storage conditions and validity period for the prepared formulation: 15-25 °C, protected from the light, available within 72 h.

According to a fixed ratio (the compound of formula (I):palonosetron free base = 872:1), the desired amount of the compound of formula (I) and palonosetron was weighed and diluted to the desired concentration to ensure that the concentration ratio of the compound of formula (I):palonosetron free base = 872:1. A low-concentration formulation for administration can be prepared by diluting a high-concentration formulation for administration with 0.02 M phosphate buffer (about pH 7.40), and the prepared formulation is used after being filtered (through a PES filter membrane). Temporary storage conditions and validity period for the prepared formulation: 15-25 °C, protected from the light, available within 72 h.

### 1.1 Administration

In the experiment, the low-dose, medium-dose, and high-dose combination groups to receive the compound of formula (I) and palonosetron were designed as (the following doses are expressed as the compound of formula (I) + palonosetron) 5 + 0.0006 mg/kg, 15 + 0.24 mg/kg, and 30 + 10 mg/kg, respectively. 5 mg/kg, 20 mg/kg, and 40 mg/kg dose groups to receive the compound of formula (I) and 10 mg/kg dose group to receive palonosetron were set, with the dose of the compound of formula (I) and the dose of palonosetron being the same as the dose of the compound of formula (I) and the dose of palonosetron in the high-dose combination group. In addition, a control group was set, wherein an equal volume of vehicle (0.02 M phosphate buffer (about pH 7.40)) was administered by intravenous injection.

Administration regimen: intravenous drip, twice a week for the first 4 weeks and once a week for the 5th week for a total of 9 times, interruption for recovery for 4 weeks after the last administration, administration time: 15 min/animal/time, and administration volume: 20 mL/kg.

1.2 The body weight and food intake were determined by observing the animal activity; hematology, blood biochemistry, urine routine, ophthalmology, and bone marrow smear examinations were performed; and the related data were analyzed.

### 1.3 Toxicokinetics

### Sampling time point:

The dose groups to receive the compound of formula (I), the dose group to receive palonosetron, and the dose groups to receive the compound of formula (I) + palonosetron: before the first and last administrations, and 2 min (± 30 s), 0.25 h (± 1 min), 0.5 h (± 1 min), 2 h (± 1 min), 4 h (± 2 min), 8 h (± 5 min), 24 h (± 30 min), and 48 h (± 30 min) after the administration;
control group: before the first and last administrations, and 2 min (± 1 min) after the administration.
Sampling method: according to the situation, before blood collection, CO₂-O₂ mixed gas (volume ratio of 7:3) can be used for anesthesia.
Sampling animals: surviving rats in each of the groups from the first and last toxicokinetic samplings.
Sampling site: the jugular vein.
Sampling amount: ≥ 0.3 mL.
Anticoagulant: EDTA-K2.
Blood sample treatment: the whole blood sample was placed in an ice box before centrifugation, and centrifuged at 2-8 °C for 10 min at the centrifugal force of 1800×g. Two tubes of plasma were isolated, wherein 50 µL, of the plasma was added to one tube, and the remaining plasma was placed in the other tube. The sample was stored at a temperature below -66 °C for detection after subpackaging.

### 1.4 Gross anatomy and histopathology examination

### 1.5 Statistical analysis

Quantitative indexes such as body weight, food intake, hematology, blood biochemistry, urine specific gravity, percentage of each cell line and megakaryocyte count by the bone marrow smear examination (if any), organ weight, and coefficient were described by mean ± standard deviation (); and qualitative indexes such as urine routine examination (except urine specific gravity) and nucleated cell proliferation by the bone marrow smear examination (if any) (binary classification, disordered multi-classification, and ordered multi-classification) were described by frequency counting. When the number of samples is less than 3, the group of data is not statistically compared.

The quantitative indexes were first subjected to homogeneity test of variance by LEVENE test. When the variance is homogenous (P > 0.05), the statistical test is performed by one-way analysis of variance (ANOVA); and when the variance is not homogeneous (P ≤ 0.05), the statistical analysis is performed by Kruskal-Wallis H rank sum test (K-W method). When the one-way analysis of variance shows that the differences are statistically significant (P ≤ 0.05), the inter-group differences are compared by Dunnett's test (Dunnett method); and when the one-way analysis of variance shows that the differences are not statistically significant (P > 0.05), the statistical analysis is completed. When the Kruskal-Wallis H rank sum test shows that the differences are statistically significant (P ≤ 0.05), the inter-group differences are compared by Mann-Whitney U test (M-W method); and when the Kruskal-Wallis H rank sum test shows that the differences are not statistically significant (P > 0.05), the statistical analysis is completed.

The ordered multi-classification indexes were analyzed by Kruskal-Wallis H rank sum test (K-W method). When the differences are statistically significant (P ≤ 0.05), the inter-group differences are compared by Dunnett's test Mann-Whitney U rank-sum test (M-W method).

The binary classification indexes were analyzed by Fisher's exact test (EXACT). When the differences are statistically significant (P ≤ 0.05), the inter-group differences are compared still by the Fisher's exact test.

Comparisons of inter-group differences were performed between each of the dose groups to receive the compound of formula (I), the dose group to receive palonosetron, and the dose groups to receive the compound of formula (I) + palonosetron, and the control group.

All tests were bilateral tests *α* = 0.05. All analyses were performed by gender. The body weight, hematology, blood biochemistry, organ weight, coefficient, and the like were statistically analyzed using the PRISTIMA version 7.2.0 data acquisition system. The other index data were statistically analyzed using Stata/IC 15.0 for Windows.

Data from general state observation, ophthalmic examination, pathological examination, and the like were subjected to descriptive analysis.

### Example 3. 29-Day Toxicity Test in Rhesus Monkeys

The compound of formula (I) was intravenously injected into rhesus monkeys 1-2 times a week for 29 consecutive days, followed by interruption for recovery for 4 weeks. The property, the degree, the dose-effect and time-effect relationships, and the reversibility of possible toxic reactions were observed, the toxic target organ or tissue is determined, and the toxicokinetic characteristics of the agent were studied to understand the relation between the exposure dose and the toxicological result in the toxicity research.

### Drug preparation

After the conversion by content, the desired amount of the compound of formula (I) was weighed and prepared to the desired concentration by adding a proper amount of 0.02 M phosphate buffer (about pH 7.40). A low-concentration formulation for administration can be obtained by diluting a high-concentration formulation for administration, and the prepared formulation is used after being filtered (through a PES filter membrane). Temporary storage conditions and validity period for the prepared formulation: 15-25 °C, protected from the light, available within 72 h.

### 1.1 Administration

In the test, the low, medium, and high doses of the compound of formula (I) tested were 2 mg/kg, 10 mg/kg, and 50 mg/kg, respectively. In addition, a control group was set, wherein an equal volume of vehicle (0.02 M phosphate buffer (about pH 7.40)) was administered in the same manner.

Administration regimen: intravenous drip, twice a week for the first 4 weeks and once a week for the 5th week for a total of 9 times, interruption for recovery for 4 weeks after the last administration, administration time: 30 min/animal/time, and administration volume: 20 mL/kg.

1.2 The body weight, food intake, body temperature (anal temperature), electrocardiogram, and blood pressure were determined by observing the animal activity; hematology, blood biochemistry, urine routine, ophthalmology, and bone marrow smear examinations were performed; and the related data were analyzed.

### 1.3 Toxicokinetics

### Sampling time point:

The dose groups to receive the compound of formula (I): before the first and last administrations, and 2 min (± 30 s), 0.25 h (± 1 min), 0.5 h (± 1 min), 2 h (± 2 min), 4 h (± 2 min), 8 h (± 2 min), 24 h (± 5 min), and 48 h (± 1 min) after the end of the administration;
control group: before the first and last administrations, and 0.25 h (± 1 min) after the administration.
Sampling animals: surviving monkeys in each of the groups.
Sampling site: saphenous vein of the lower limb or other suitable veins.
Sampling amount: ≥ 0.4 mL.
Anticoagulant: EDTA-K2.

Blood sample treatment: the whole blood sample was placed in an ice box before centrifugation, and centrifuged at 2-8 °C for 10 min at the centrifugal force of 1800×g. Two tubes of plasma were isolated, wherein 50 µL, of the plasma was added to one tube, and the remaining plasma was placed in the other tube. The sample was stored at a temperature below -66 °C for detection after subpackaging.

### 1.4 Gross anatomy and histopathology examination

### 1.5 Statistical analysis

Quantitative indexes such as body weight, food intake, body temperature, electrocardiogram, blood pressure, hematology, blood biochemistry, urine specific gravity, percentage of each cell line and megakaryocyte count by the bone marrow smear examination (if any), organ weight, and coefficient were described by mean ± standard deviation (*X̅*±*SD*); and qualitative indexes such as nucleated cell proliferation by the bone marrow smear examination (if any) and urine routine examination (except urine specific gravity) (binary classification, disordered multi-classification, and ordered multi-classification) were described by frequency counting. When the number of samples is less than 3, the group of data is not statistically compared.

The quantitative indexes were first subjected to homogeneity test of variance by LEVENE test. When the variance is homogenous (*P* > 0.05), the statistical test is performed by one-way analysis of variance (ANOVA); and when the variance is not homogeneous (*P* ≤ 0.05), the statistical analysis is performed by Kruskal-Wallis H rank sum test (K-W method). When the one-way analysis of variance shows that the differences are statistically significant (*P* ≤ 0.05), the inter-group differences are compared by Dunnett's test (Dunnett method); and when the one-way analysis of variance shows that the differences are not statistically significant (*P* > 0.05), the statistical analysis is completed. When the Kruskal-Wallis H rank sum test shows that the differences are statistically significant (*P* ≤ 0.05), the inter-group differences are compared by Mann-Whitney U test (M-W method); and when the Kruskal-Wallis H rank sum test shows that the differences are not statistically significant (*P* > 0.05), the statistical analysis is completed.

The ordered multi-classification indexes such as urine examination (clarity, glucose, bilirubin, ketone body, occult blood, pH value, protein, urobilinogen, and white blood cell) and the nucleated cell proliferation by the bone marrow smear were analyzed by Kruskal-Wallis H rank sum test (K-W method). When the differences are statistically significant (*P* ≤ 0.05), the inter-group differences are compared by Mann-Whitney U test (M-W method).

The binary classification (nitrite) and disordered multi-classification (urine color) indexes were analyzed by Fisher's exact test (EXACT). When the differences are statistically significant (*P* ≤ 0.05), the inter-group differences are compared still by the Fisher's exact test.

Comparisons of inter-group differences were performed between each of the dose groups to receive the compound of formula (I) and the control group.

All tests were bilateral tests *α* = 0.05. All analyses were performed by gender. The body weight, food intake, hematology, blood biochemistry, organ weight, coefficient, and the like were statistically analyzed using the PRISTIMA version 7.2.0 data acquisition system. The other indexes were statistically analyzed using Stata/IC 15.0 for Windows. Data from general state observation, ophthalmic examination, pathological examination, and the like were subjected to descriptive analysis.

### Test Example 1. Water Solubility Data and Chemical Stability

### 1.1 Preparation of reagent

Reagent: NaH₂PO₄·2H₂O

### 1.2 Preparation method

The preparation according to 100 mL specification is as follows:
pH = 3.0: phosphate buffer: 100 mL of 2 mmol/L NaH₂PO₄, adjusted to pH 3.0 with 0.1 M H₃PO₄.
pH = 4.0: phosphate buffer: 100 mL of 2 mmol/L NaH₂PO₄, adjusted to pH 4.0 with 0.1 M H₃PO₄.
pH = 7.0: ultra-pure water.
pH = 9.0: phosphate buffer: 100 mL of 2 mmol/L Na2HPO₄, adjusted to pH 9.0 with 0.1 M NaOH.

### 1.3 Test method

A proper amount of a test compound was weighed, and the solution was added in small batches. The mixture was stirred until the test compound was dissolved. The content of the compound in the solution was determined. The data are shown in Table 1.

### 2.1 Stability test of compound

1 mg of the sample was weighed and added to a vial. The vial was placed in a vacuum bag, which was vacuumized and placed in a container filled with allochroic silica gel. The container was sealed. Two parts were prepared in parallel. Adequate parts were prepared according to sampling time points, and the two parts for each sampling time point were placed at 4 °C and room temperature, respectively. The solubility of the compound of formula (I) was determined at different pH values. The data are shown in Table 1.

**Table 1**

| pH | Solubility | Saturated solubility |
|---|---|---|
| 7.4 | 26mg/mL | 19.8mg/mL |
| 9.0 | 28 mg/mL | 21.4 mg/mL |

| | | |
|---|---|---|
| Note: good: after storage for 7 days, the purity is reduced by less than 0.5%; medium: after storage for 7 days, the purity is reduced by 0.5%-2.0%; and poor: after storage for 7 days, the purity is reduced by more than 2.0%. | | |

### Test Example 2. Hemolytic Effect

10 mL of red blood cells (RBCs) were randomly collected from the jugular vein or central auricular artery of a rabbit (EDTA whole blood), placed in a conical flask with glass beads, and shaken for 10 min to remove fibrinogen, resulting in defibrinated blood. 10 volumes of sodium chloride injection were added. The mixture was shaken uniformly and centrifuged at 1500 rpm for 10 min, and the supernatant was removed. The precipitated red blood cells were washed 3 times with sodium chloride injection according to the above method until the supernatant did not appear red. The obtained red blood cells were prepared into 2% (v/v) suspension with sodium chloride injection for later use.

A test sample (the compound of formula (I)) was dissolved in PBS (pH 7.4 or pH 5), and the solution was filtered and prepared to the concentrations of 0.4 mg/mL, 0.8 mg/mL, 1.2 mg/mL, 1.6 mg/mL, and 2 mg/mL for later use.

A certain amount of the test sample solution was added to the above hemoglobin for testing in the supernatant.

If the solution in the test tube is clear and red and no cells or a small amount of red blood cells remain at the bottom of the tube, it indicates that hemolysis has occurred; and if all of the red blood cells are sedimented and the supernatant is colorless and clear, it indicates that no hemolysis has occurred. If there is a brownish-red or reddish-brown flocculent precipitate in the solution and it is not dispersed even after 3-5 times of gentle inversion, it indicates that red blood cell coagulation may have occurred. A further observation should be performed under a microscope, and if red blood cell aggregation is visible, it indicates that coagulation has occurred. The hemolytic effect of the compound provided by the present disclosure was determined by this method.
Conclusion: the compound of formula (I) shows no hemolytic effect even at a concentration up to 2 mg/mL.

### Test Example 3. Hemolytic Effect of Rolapitant Emulsion

Rolapitant emulsion (formula: 4.4% polyethylene glycol 15-hydroxystearate, 1.1% medium chain triglyceride, and 0.66% soybean oil) was prepared by reference to the method described in CN102573475, and prepared with PBS to concentrations of 0.18 mg/mL, 0.09 mg/mL, 0.045 mg/mL, 0.023 mg/mL, 0.011 mg/mL, 0.056 mg/mL, and 0.028 mg/mL for later use.

The hemolytic effect was determined by the method described in Test Example 2.

Conclusion: All concentrations of rolapitant emulsion have the hemolytic effect.

### Test Example 4: Pharmacokinetic Test in Cynomolgus Monkeys

With cynomolgus monkeys as test animals, the plasma concentrations of the compound of formula (I) at different time points after administration by injection were determined by LC/MS/MS. The pharmacokinetic performance of the compound was studied in cynomolgus monkeys and its pharmacokinetic characteristics were evaluated.

### Drug preparation

A certain amount of the test compound was weighed and prepared into a solution at pH 4.0 with 20 mmol/L sodium dihydrogen phosphate for later use.

### 1.1 Administration

Intravenous drip, injection time: about 30min, administration dose: 3.54 mg/kg, administration concentration: 2 mg/mL, and administration volume: 5 mL/kg.

### 1.2 Operation

Blood was collected from femoral vein before administration and 5 min, 0.25 h, 0.5 h, 1 h, 2 h, 4 h, 6 h, 8 h, 10 h and 24 h after administration, about 0.6 mL for each sample. The blood samples were anticoagulated with sodium heparin and placed on ice immediately after collection. The blood samples were placed in marked centrifuge tubes after collection, and plasma was isolated by centrifugation (centrifugation conditions: 2200 g of centrifugal force, 10 min, 2-8 °C).

The contents of the compound of formula (I) and rolapitant in the plasma samples were determined by LC/MS/MS.

### 1.3 Pharmacokinetic parameters

**Table 2**

| | Compound | |
|---|---|---|
| | (ng/mL)^{a} | (ng/mL)^{b} |
| AUC₀₋₂₄ₕ (ng/mL^{∗}h) | 1434.78 | 8410.94 |
| T_{1/2}(h) | 0.47 | 13.16 |
| MRT 0-∞ (h) | 0.26 | 8.17 |

| | | |
|---|---|---|
| Note: a: pharmacokinetic parameters of the compound of formula (I) in cynomolgus monkeys; and b: pharmacokinetic parameters of rolapitant by the metabolism of the compound of formula (I) in cynomolgus monkeys. | | |

Conclusion: in the study on the pharmacokinetics of the compound of formula (I) in cynomolgus monkeys, most of the compounds are quickly converted into the active metabolite rolapitant in cynomolgus monkeys, which has good pharmacokinetic properties.

## Claims

1. Use of a compound of formula (I) or a pharmaceutically acceptable salt thereof in combination with a 5-HT3 receptor antagonist in the preparation of a medicament for preventing or treating nausea and/or vomiting,

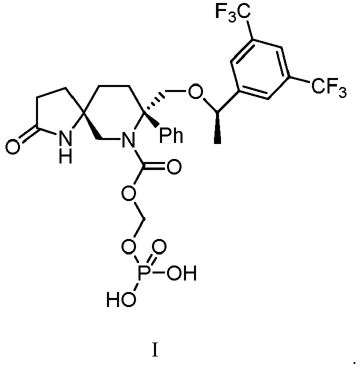

2. The use according to claim 1, wherein the 5-HT3 receptor antagonist is selected from the group consisting of granisetron, ondansetron, ramosetron, tropisetron, palonosetron, and dolasetron, preferably palonosetron.

3. The use according to claim 1 or 2, wherein the compound of formula (I) or the pharmaceutically acceptable salt thereof is administered at a dose of 10-500 mg.

4. The use according to any one of claims 1-3 wherein the 5-HT3 receptor antagonist is administered at a dose of 0.075-1 mg.

5. The use according to any one of claims 1-4, wherein the compound of formula (I) or the pharmaceutically acceptable salt thereof is administered at a frequency selected from the group consisting of once a day, twice a day, three times a day, once a week, and once every two weeks.

6. The use according to any one of claims 1-5, wherein the 5-HT3 receptor antagonist is administered at a frequency selected from the group consisting of once a day, twice a day, three times a day, once a week, and once every two weeks.

7. The use according to any one of claims 1-6, wherein the nausea and/or vomiting is selected from the group consisting of chemotherapy-induced nausea and vomiting, radiotherapy-induced nausea and vomiting, and postoperative nausea and vomiting.

8. The use according to any of claims 1-7, wherein the nausea and/or vomiting is selected from the group consisting of nausea and/or vomiting during the acute phase and nausea and/or vomiting during the delayed phase.

9. A pharmaceutical composition comprising a compound of formula (I) or a pharmaceutically acceptable salt thereof, a 5-HT3 receptor antagonist, and one or more pharmaceutically acceptable carriers.

10. A pharmaceutical package, wherein a composition comprising a compound of formula (I) or a pharmaceutical salt thereof and a 5-HT3 receptor antagonist is packaged.

11. A method for preventing or treating nausea and/or vomiting, comprising administering to a patient a compound of formula (I) or a pharmaceutically acceptable salt thereof and a 5-HT3 receptor antagonist.

12. The method according to claim 11, wherein the compound of formula (I) or the pharmaceutically acceptable salt thereof and the 5-HT3 receptor antagonist are administered after the occurrence of a vomiting-inducing event or no more than 1 or 2 h before the occurrence of a vomiting-inducing event.
